# DEMANDE DE BREVET EUROPEEN

(11) **EP 0 820 780 A1**
(43) Date de publication de la demande: **28.01.1998**
(21) Numéro de dépôt: 97401771.7
(22) Date de dépôt: 23.07.1997
(51) Int. Cl.: A61M 15/00

(54) **Procédé de vaporisation et appareils d'inhalation le mettant en oeuvre**

(30) Priorité: 23.07.1996 FR 9609217
(71) Demandeur: Pedrali, Frank, 74000 Annecy (FR); Mayet Pierre de Bresse (SA), 71270 Pierre de Bresse (FR)
(72) Inventeur: Grandmottet, Claude, 71270 Pierre de Bresse (FR); Pedrali, Franck, 74000 Annecy (FR)
(74) Mandataire: Bruder, Michel

(57) **Abrégé**

La présente invention concerne un procédé pour l'amélioration de la diffusion d'un produit bronchodilatateur sous forme d'aérosol distribué dose par dose en "spray", ainsi qu'un appareil (1) apte à mettre en oeuvre le procédé pour inhaler ou insuffler par voie buccale, nasale ou autre, ledit produit (2) en aérosol (3) auto-doseur dont chaque dose est distribuée en "spray", c'est-à-dire vaporisée grâce à un vecteur-propulseur à gaz à l'intérieur d'une chambre d'expansion (5) couplée à un embout (16) buccal, nasal, auditif ou autre, par l'intermédiaire d'un clapet d'étanchéité (17), ledit appareil (1) comportant des moyens de chauffage *caractérisé* en ce que l'organe chauffant (10) des moyens de chauffage est disposé pour réchauffer l'intérieur de la chambre d'expansion (5) en association avec une source d'énergie (7) avantageusement autonome et contrôlée pour activer ledit organe chauffant (10) au moins en présence d'une dose de produit (2) dans le but de le réchauffer jusqu'à une température pré-déterminée. L'appareil (1) est particulièrement applicable pour le traitement de sujets asthmatiques ou bronchopathiques.

## Description

La présente invention concerne un procédé d'inhalation ou d'insufflation par voie buccale, nasale ou analogues d'un produit sous forme d'aérosol distribué dose par dose en "spray" c'est-à-dire vaporisé grâce à un vecteur-propulseur à gaz, engendrant pour la plupart des principes actifs utilisés à ce jour, une très spectaculaire amélioration de leur diffusion et, par là, de leur efficacité clinique en matière de pneumologie et d'oto-rhino-laryngologie. L'invention concerne également un appareil apte à la mise en oeuvre d'un tel procédé et son application particulière pour le soulagement de sujets asthmatiques ou bronchopathiques.

Dans le contexte actuel de réduction des coûts sociaux et de la santé, seuls les grands groupes pharmaceutiques possèdent encore les moyens technologiques et financiers pour développer de nouvelles molécules originales ; c'est l'une des raisons de l'évolution de la recherche et développement dans l'industrie pharmaceutique vers la galénique c'est-à-dire, non plus vers la recherche de nouvelles molécules, mais vers des formes de prise du médicament pour lesquelles le support ou le vecteur du principe actif doit subir certaines modifications afin de mieux contrôler les doses distribuées, d'améliorer sa bio-disponibilité dans l'organisme, de réduire sa posologie ou encore d'atténuer les éventuels effets secondaires et naturellement d'en réduire le prix.

Dans le domaine particulier de la pneumologie et de l'oto-rhino-laryngologie on peut aujourd'hui distinguer deux grandes catégories d'administration des médicaments dans les thérapies respiratoires :
- les aérosols définis selon le codex français comme des brouillards de particules solides ou liquides en dispersion dans l'air atmosphérique ou dans un gaz et suffisamment fines pour y rester en suspension,
- les inhalateurs à poudre sèche dont le développement est plus récent et qui représente à l'heure actuelle le mode d'administration des anti-asthmatiques les plus en pointe ; il s'agit en fait d'un dispositif mécanique délivrant une dose de principe actif micronisé moyennant une double opération de chargement et de fortes inspirations du patient à travers un embout.

Cette technologie récente est d'ores et déjà contestée dans la mesure où il est établi que la pénétration des poudres micronisées restent limitée dans l'arbre bronchique au niveau des troncs supérieurs et des voies aériennes supérieures. En outre, du côté des utilisateurs, ceux-ci ne ressentent pas la prise d'un médicament ainsi distribué, ce qui a incontestablement pour effet de réduire l'action placebo de l'acte thérapeutique. En revanche, il est certain que cette technologie procure une plus grande maîtrise des doses distribuées et écarte toutes éventuelles irritations des muqueuses liées au gaz vecteur des aérosols.

On a pu également reprocher au distributeur d'aérosol une nécessaire synchronisation entre la pression à exercer sur le distributeur et l'inhalation du produit qui est souvent délicate.

En l'état actuel des comparaisons cliniques entre les technologies concurrentes on peut dire que tant les médecins que les patients sont à ce jour tout à fait partagés et aucun résultat d'expertise n'a pu aujourd'hui conclure sérieusement à une supériorité des formes en poudre par rapport aux formes en aérosol délivrées en "spray".

Ceci est d'autant plus accentué que tous les aérosols distribués en "spray" connus et utilisés à ce jour, utilisent des gaz-vecteurs qui ont pour effet d'abaisser la température du produit délivré par rapport à l'air ambiant, en application directe de la loi des gaz parfaits. Or on sait bien que le froid est un bronchoconstricteur ce qui créé une situation tout à fait paradoxale dans la mesure où on veut véhiculer un produit à visée bronchodilatatrice avec un vecteur bronchoconstricteur diminuant à tout le moins l'efficacité recherchée.

La présente invention qui entre dans la catégorie des produits sous forme d'aérosol distribués en "spray" vise précisément à remédier à tous les inconvénients précités.

A cet égard, il est proposé conformément à l'invention, un procédé d'inhalation ou d'insufflation par voie buccale, nasale ou autre, d'un produit sous forme d'aérosol distribué dose par dose en "spray" c'est-à-dire vaporisé grâce à un vecteur-propulseur à gaz notamment utilisable pour le soulagement d'un utilisateur par exemple asthmatique ou bronchopathique caractérisé en ce que l'on réchauffe à une température déterminée chaque dose de produit ainsi délivrée avant de l'inhaler ou de l'insuffler.

Selon un tel procédé on est parvenu à une efficacité clinique supérieure de 30 à 40 % à toutes les autres formes y compris d'ailleurs les formes en poudre, et ce, à posologie égale, par la mesure de la réversibilité obtenue avec les principales molécules bronchodilatatrices utilisées dans le monde au niveau des troncs moyens et périphériques bronchiques ; or, c'est précisément à ce niveau que l'on obtient, outre le soulagement du patient, une réelle amélioration dans le temps de l'état de base du sujet asthmatique ou bronchopathique avec la conséquence heureuse d'améliorer le pronostic de la maladie à long terme.

Selon une caractéristique fondamentale du procédé conforme à l'invention, il est prévu que l'on maintienne dans une chambre d'expansion, une dose déterminée de produit médicamenteux, soit pendant un temps pré-déterminé pour que le même produit atteigne une température optimale d'inhalation ou d'insufflation proche de la température du corps humain, c'est-à-dire de l'ordre de 37°C soit que l'on contrôle la température du produit à l'intérieur de la même chambre d'expansion pour ne l'inhaler ou l'insuffler que lorsqu'il aura atteind la même température pré-déterminée, avantageusement fixée à 37°C particulièrement lorsqu'il s'agit de produit à visée bronchodilatatrice.

Pour la mise en oeuvre d'un tel procédé, il a été suggéré conformément à un autre objet de l'invention un appareil pour inhaler ou insuffler par voie buccale, nasale ou autre, un produit en aérosol auto-doseur dont chaque dose est distribuée en "spray" c'est-à-dire vaporisée grâce à un vecteur-propulseur à gaz à l'intérieur d'une chambre d'expansion couplée à un embout buccal, nasal, auditif ou autre, par l'intermédiaire d'un clapet d'étanchéité, ledit appareil comportant des moyens de chauffage caractérisé en ce que l'élément chauffant des moyens de chauffage est disposé pour réchauffer l'intérieur de la chambre d'expansion en association avec une source d'énergie avantageusement autonome et contrôlée pour activer ledit élément chauffant, au moins en présence d'une dose de produit, dans le but de le réchauffer jusqu'à une température pré-déterminée.

A cet effet, on connaît déjà de nombreux types d'appareils consistant tous à délivrer le principe actif soit directement par simple pression sur un éjecteur, soit par l'intermédiaire d'une chambrette d'expansion pour amoindrir les effets de pression sur les muqueuses, ou encore par des appareils avec détendeur déclenchés par aspiration, par exemple.

Aucun de ces dispositifs ne sont aujourd'hui équipés d'un réchauffage produit avant inhalation ou insufflation.

D'un autre côté, on connaît un certain nombre d'inhalateurs portables comprenant des moyens de réchauffage alimentés par une source d'énergie autonome, c'est le cas par exemple de l'inhalateur décrit dans le brevet français 93.00825 qui se caractérise en ce qu'il comprend dans le circuit d'air aspiré par le nez du patient, un disque en matière absorbante imprégné d'un produit inhalant thérapeutique, de telle manière que l'air ainsi inspiré soit à température sensiblement ambiante. En outre, l'élévation même légère de température du vecteur air contribue à une meilleure diffusion thérapeutique du produit d'imprégnation.

On connaît également un dispositif ressortant du brevet américain US-4.735.217 portant à une température proche des fumées de cigarettes, un flux d'air inhalé par la bouche, porteur de produit telle que la nicotine, destiné à désaccoutumer les gros fumeurs. Ce dispositif inhalateur chauffant est, comme le précédent, destiné à porter le vecteur air à une température adéquate en le faisant circuler autour d'un élément chauffant avant d'être inhalé.

Plus généralement, il n'a jamais été décrit et pas davantage suggéré un dispositif permettant de réchauffer des produits sous forme d'aérosol distribués en "spray" par l'intermédiaire d'une zone d'expansion intercalée entre l'aérosol et l'utilisateur, aux fins de réchauffer le produit en donnant à l'utilisateur le moyen de contrôler qu'il est à bonne température, selon plusieurs variantes de l'invention qui seront décrites plus loin.

D'autres caractéristiques et avantages ressortiront mieux encore dans la description de variantes préférées de l'appareil selon l'invention données ci-après à titre d'exemples non limitatifs en référence aux dessins dans lesquels :
- la figure 1 représente en élévation et en coupe selon le plan vertical de symétrie l'appareil conforme à l'invention dans une variante comportant un embout buccal,
- la figure 2 est une vue de côté de l'appareil de la figure 1, montrant l'embout de face.
- la figure 3 est une vue de dessus en coupe de l'appareil selon un plan de coupe III-III de la figure 1.

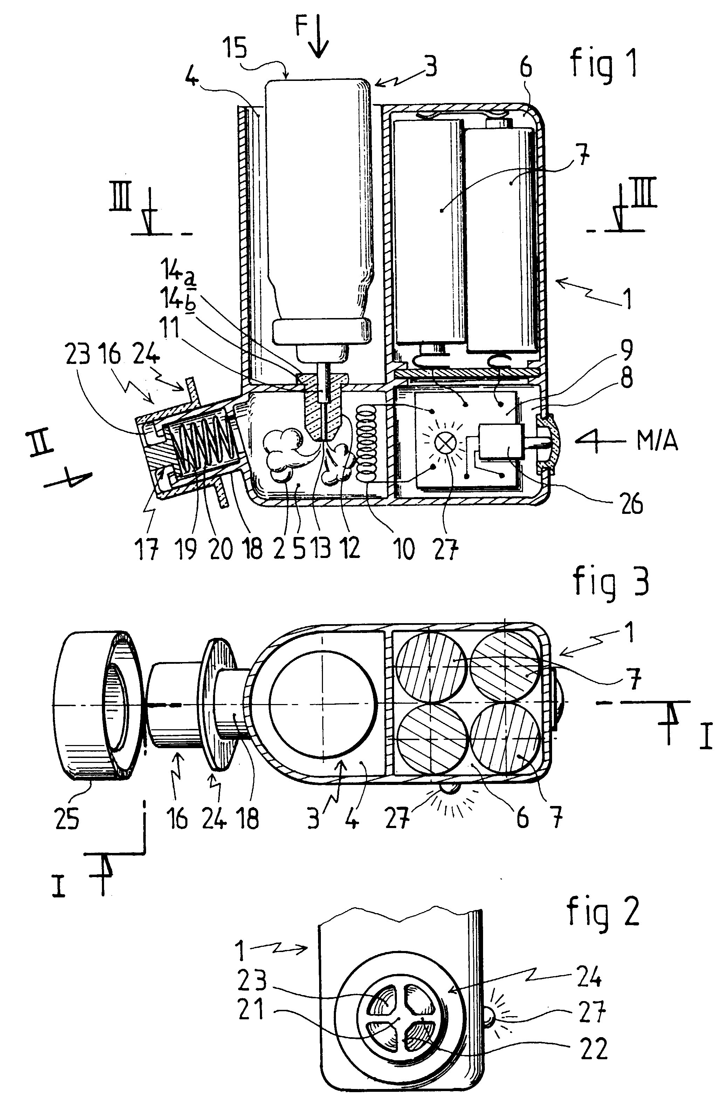

En référence aux figures, l'appareil 1 pour inhaler ou insuffler par voie buccale, nasale ou autres, un produit 2 en aérosol auto-doseur 3 comprenant un premier réceptacle 4 ouvert vers le haut pour recevoir verticalement la plupart des aérosols-doseurs 3, un second réceptacle dans le prolongement vertical du premier servant de chambre d'expansion 5 pour le produit 2, jouxtant un troisième réceptacle 6 comportant la source d'énergie 7, et disposé au droit du précédent et de préférence à côté de la chambre d'expansion 5, un réceptacle 8 comportant les éléments de contrôle et de régulation 9 d'un organe de chauffage 10 monté dans la chambre 5 sous forme d'une résistance spiralée telle que représentée à titre d'exemple sur la figure 1. Muni de ces quatres réceptacles, 4,5,6 et 8, avantageusement disposés comme représenté en figures 1 et 3, l'appareil 1 est de dimension telle qu'il soit aisément manipulable par l'utilisateur et suffisamment compact pour être transporter sans gène particulière par celui-ci ; on notera accessoirement que l'encombrement de l'appareil 1 est surtout lié à la taille de l'aérosol 3 mais également à l'encombrement de la nécessaire source autonome d'énergie 7 normalement réalisée à partir de quatre piles sèches ou batteries du type LR6, susceptibles de fournir une puissance électrique suffisante à l'organe de chauffage 10. Conformément aux procédés d'inhalation ou d'insufflation caractérisant l'invention, le produit 2 provenant de l'aérosol 3 est introduit dans la chambre d'expansion 5, dose par dose, chacune correspondant classiquement à une pression axiale du tube éjecteur 11 pénétrant à l'intérieur de l'aérosol 3 pour libérer la quantité de principe actif prévue par le laboratoire fabriquant le principe actif de l'aérosol.

Naturellement, dans l'appareil 1 l'aérosol 3 fonctionne à l'inverse c'est-à-dire qu'il est disposé tête en bas à l'intérieur du réceptacle 4 de telle manière que le tube éjecteur 11 vienne s'enfiler axialement dans une pièce de révolution 12 constituant un adaptateur et un élément d'étanchéité en une matière souple, avantageusement en caoutchouc, comportant axialement un canal 13 débouchant susceptible de mettre en communication l'aérosol 3 avec la chambre d'expansion 5. Le joint adaptateur 12 comprend, en outre, un premier épaulement 14a procurant l'étanchéité entre le réceptacle 4 et la chambre 5 ; de même l'adaptateur 12 est muni à peu près à mi-hauteur d'un second épaulement 14b sur lequel vient reposer l'extrémité du tube éjecteur 11 de l'aérosol 3 ; ainsi et dans la mesure où l'aérosol 3 est accessible par sa face inférieure 15 située normalement en partie supérieure de l'appareil 1, de préférence dépassant légèrement du réceptacle 4 afin de le rendre accessible à un doigt de l'utilisateur qui par pression verticale F pourra comprimer le tube éjecteur 11 sur l'épaulement de retenue 14b du joint 12 et libérer ainsi par le canal 13 une dose de produit 2 délivrée en "spray" c'est-à-dire vaporisé grâce à un vecteur propulseur à gaz.

Accessoirement on notera que le joint adaptateur 12 est prévu pour pouvoir recevoir les tubes éjecteurs 11 de la plupart des aérosols du commerce 3, c'est-à-dire que le diamètre du canal 13 traversant le joint 12 peut être largement étiré radialement pour s'adapter à tous les diamètres de tubes éjecteurs 11, tout en maintenant pour les plus fins une étanchéité latérale suffisante.

Une fois le produit 2 à l'intérieur de la chambre 5, il y est maintenu en suspension dans la mesure où son éjection n'est possible que par un embout 16 normalement fermé au repos par un clapet 17. Parallèlement et comme il sera dit plus loin, l'organe chauffant 10 est actionné par les circuits d'alimentation et de contrôle 6,9 de telle manière que le produit 2 enfermé dans la chambre d'expansion 5 puisse être réchauffé à une température pré-déterminée grâce à des moyens de régulation qui seront examinés plus loin.

Pour les raisons largement évoquées en préambule, le produit 2 distribué en "spray" à partir de l'aérosol 3 s'étend dans la chambre 5 dont le volume intérieur est normalement compris entre 20 et 50 cm³ pour être utilisable avec la plupart des aérosols du commerce. L'organe de chauffage 10, par exemple une grille ou un fil spiralé, permet une augmentation de température du produit 2 jusqu'à environ 37°C, température considérée comme optimale, conformément au procédé selon l'invention ; bien entendu, cet organe de chauffage 10, associé conventionnellement à un circuit de régulation peut être remplacé par tout autre dispositif permettant au produit 2 d'atteindre la température requise. Il peut notamment s'agir, sans sortir de l'invention, de produits tel que réacteur chimique biocompatible, qui parait néanmoins une solution peu adaptée aux caractéristiques et aux buts de l'appareil 1 ; il peut également s'agir de produits tels qu'éléments chauffants à coefficient de température positive consistant en des plaquettes issues de céramiques PTC que connaît bien l'Homme de l'Art. Après mise sous tension manuelle l'appareil 1, il est alors possible d'obtenir une auto-régulation du chauffage de la chambre 5 puisque la résistance de telles céramiques augmente avec la température, ce qui permet, en choisissant bien les caractéristiques techniques de l'élément chauffant 10, de prévoir un chauffage de la chambre auto-régulé aux alentours de 37°C par variation de la résistance de l'élément chauffant ; il suffira simplement de mettre l'appareil hors tension dès qu'on ne l'utilisera plus.

Il est à noter ici que de tels dispositifs optionnels sont aujourd'hui encore coûteux et on décrira plus loin le système préféré de régulation de l'appareil 1.

Lorsque la température adéquate du produit 2 dans la chambre 5 est atteinte, l'utilisateur est à même de pouvoir inhaler le produit grâce à l'embout 16 qui peut naturellement être conformée pour une inhalation par la bouche ou par le nez selon des variantes que l'Homme du Métier saura parfaitement adapter sans faire preuve d'Activité Inventive. On notera d'ailleurs ici qu'un tel embout 16 pourra avoir toutes les formes requises pour permettre non plus seulement des inhalations buccales ou nasales mais des insufflations pour injecter le produit 2, réchauffé dans la chambre 5, dans toute partie du corps qui sont habituellement traitées par les aérosols du commerce (par exemple insufflation par voie auditive, etc ...).

Selon la variante préférée de l'invention et en référence à la figure 1 il sera maintenant décrit la forme particulière de l'embout buccal 16 muni de son clapet 17.

La chambre 5 est reliée à l'embout 16 via un tube de sortie 18 comportant dans sa partie d'extrémité proximale une restriction de diamètre servant intérieurement de siège pour une soupape 19 maintenue sur ce siège par un ressort 20, d'un côté en appui sur l'appareil 1 et de l'autre sur la face interne de la soupape 19.

Coulissant sur la partie externe du tube de sortie 18 de la chambre 5, l'embout buccal 16 peut entraîner dans son coulissement axial la soupape 19 grâce à une pièce d'entraînement avantageusement circulaire 21 disposée à son extrémité proximale et solidarisée en son centre à la soupape 19 pour son entraînement axial grâce à des bras radiaux 22 déterminant dans la même pièce des orifices 23 par lesquels peut s'évacuer le produit 2 enfermé dans la chambre 5 dès lors que la soupape 19 est repoussée, libérant ainsi l'extrémité supérieure du tube 18 et créant le passage nécessaire au produit 2 au travers des orifices 23, pour éjecter finalement le produit 2 dans la bouche de l'utilisateur. On notera qu'accessoirement et avantageusement une collerette extérieure 24 disposée à l'autre extrémité distale de l'embout 16 procure un élément d'appui pour les lèvres de l'utilisateur, facilitant ainsi le mouvement axial pour ouvrir le clapet 17 et inhaler par conséquent le produit 2 réchauffé dans la chambre d'expansion 5.

Un capot 25 viendra avantageusement coiffer l'embout 16 lorsque l'appareil 1 ne sera pas utilisé et ce pour une bonne aseptie de l'ensemble.

Selon une première variante de l'appareil 1 conforme à l'invention, le circuit de régulation 9 permet d'alimenter l'organe de chauffage 10 de la chambre 5 pendant un temps T susceptible de porter le produit 2 en suspension dans la chambre 5, à sensiblement la température pré-déterminée, en principe 37°C. Selon l'exemple de la description et pour une chambre d'expansion 5 de 20 cm³ il est prévu d'alimenter une résistance spiralée alimentée sous 4,5 volts, et de la laisser sous tension environ 10 secondes. Dans ce cas le circuit 9 est un circuit permettant la mise sous tension de la résistance 10 à partir de la source d'énergie 7 et le maintient en l'état pendant un temps déterminé au delà duquel la source 7 est mise automatiquement hors tension. Bien entendu, la mise sous tension du circuit d'alimentation 9 peut s'effectuer soit manuellement par l'utilisateur lui-même en utilisant un bouton M/A par exemple disposé à l'arrière de l'appareil comme en figure 1, de telle manière que par une pression sur une zone d'affaiblissement du boîtier de l'appareil un interrupteur 26 puisse être actionné, le circuit 9 prenant en compte l'ordre de commutation pendant le temps requis, par exemple 10 secondes, au delà duquel l'interrupteur revient automatiquement en position ouvert. Naturellement, l'utilisateur préalablement à cette opération aura introduit une dose de produit 2 à l'intérieur de la chambre 5 par pression sur l'aérosol 3 (flèche F de la figure 1) ; afin de faciliter le fonctionnement général qui vient d'être décrit, il est prévu de coupler au dispositif de contrôle 9 un système visuel indiquant qu'il est sous tension, en adjoignant par exemple une led 27 visible à l'extérieur du boîtier par l'utilisateur ; ainsi lorsque ce dernier appuiera sur l'interrupteur 26 la led 27 restera allumée pendant le temps requis permettant de parvenir à la température voulue à l'intérieur de la chambre 5, c'est-à-dire que lorsque la led 27 se sera éteinte, l'utilisateur pourra à ce moment là actionner le clapet 17 grâce à l'embout 16 préalablement mis en bouche.

Selon une autre variante plus avantageuse de l'invention (et non représentée sur les figures) on peut coupler l'interrupteur 26 et la pression suivant F sur l'aérosol 3, injectant du même coup le produit 2 dans la chambre 5 et mettant simultanément en route le circuit de régulation 9 ; il suffira à l'utilisateur d'attendre l'extinction de la led 27 pour inhaler, comme il a été dit, le produit 2 grâce à l'embout buccal 16.

Selon encore une autre variante de l'invention, le circuit de régulation 9 peut être un simple circuit de contrôle de la température à l'intérieur de la chambre 5 qui lorsque celle-ci atteint le seuil requis (37°C), arrête automatiquement l'alimentation de l'organe chauffant 10. Dans ce cas, il suffit d'une simple mise en marche de l'appareil soit par action sur l'interrupteur 26 comme il a été dit précédemment, soit par pression sur la partie arrière 15 de l'aérosol 3 (flèche F) ; la led 27 indiquera que le dispositif est sous tension et elle ne s'éteindra que lorsque la température aura atteint les 37°C à l'intérieur de la chambre 5 ; on observera qu'il est tout à fait possible de prévoir un capteur de température permettant de réaliser de façon très classique un tel dispositif de contrôle et de régulation thermique.

Enfin et accessoirement, il peut être utilisé non pas un système visuel telle que la led 27, mais un système auditif (non représenté sur les dessins) permettant de prévenir l'utilisateur par un bip convenable, que l'appareil 1 est prêt à l'emploi ; bien entendu, un couplage des deux dispositifs visuel et auditif constituera une variante particulièrement avantageuse.

Quelle que soit la variante de l'appareil 1, celui-ci apparait désormais comme particulièrement adapté à délivrer des produits à visée bronchodilatatrice sous la forme de "spray" pour le soulagement de sujets asthmatiques ou bronchopathiques.

L'appareil ainsi décrit ne créé aucune irritation des muqueuses et on observe une parfaite tolérance des produits dans la mesure où par leurs réchauffages avant inhalation ou insufflation, on supprime le choc thermique et on obtient une exceptionnelle qualité de diffusion puisque les effets nocifs du vecteur gaz bronchoconstricteur ont maintenant disparu.

## Revendications

1. Procédé pour l'amélioration de la diffusion d'un produit sous forme d'aérosol distribué dose par dose en "spray" c'est-à-dire vaporisé, grâce à un vecteur-propulseur à gaz **caractérisé** en ce que l'on réchauffe à une température pré-déterminée de l'ordre de 37°C chaque dose de produit ainsi délivrée avant de l'inhaler ou de l'insuffler.

2. Procédé selon la revendication 1 **caractérisé** en ce que l'on maintient dans une chambre d'expansion chaque dose de produit délivrée pendant un temps pré-déterminé suffisant pour atteindre la température pré-déterminée.

3. Procédé selon la revendication 2 **caractérisé** en ce que le temps de maintien des doses de produit dans la chambre d'expansion est programmé dès l'admission d'une dose dans la chambre, des moyens d'alerte sonores et/ou visuels indiquant que la température requise est atteinte.

4. Appareil (1) apte à mettre en oeuvre le procédé, selon l'une quelconque des revendications 1 à 3 précédentes pour inhaler ou insuffler par voie buccale, nasale ou autre un produit (2) en aérosol (3) auto-doseur dont chaque dose est distribuée en "spray", c'est-à-dire vaporisée grâce à un vecteur-propulseur à gaz à l'intérieur d'une chambre d'expansion (5) couplée à un embout (16) buccal, nasal, auditif ou autre, par l'intermédiaire d'un clapet d'étanchéité (17), ledit appareil (1) comportant des moyens de chauffage **caractérisé** en ce que l'organe chauffant (10) des moyens de chauffage est disposé pour réchauffer l'intérieur de la chambre d'expansion (5) en association avec une source d'énergie (7) avantageusement autonome et contrôlée pour activer ledit organe chauffant (10) au moins en présence d'une dose de produit (2) dans le but de le réchauffer jusqu'à une température pré-déterminée de l'ordre de 37°C.

5. Appareil (1) selon la revendication 4 **caractérisé** en ce que l'organe chauffant (10) est du type d'une résistance disposée sur les parois ou à l'intérieur de la chambre d'expansion (5).

6. Appareil (1) selon la revendication 5 **caractérisé** en ce que la résistance a la forme d'une plaque, d'une grille ou d'un fil avantageusement spiralé.

7. Appareil (1) selon l'une quelconque des revendications 5 ou 6 **caractérisé** en ce que la résistance est alimentée par un circuit de régulation (9) associé à une source d'énergie autonome (7) telle que pile ou batterie, prévu pour maintenir la résistance sous tension pendant un temps déterminé à compter de l'instant où une dose de produit (2) est dans la chambre (5).

8. Appareil (1) selon l'une quelconque des revendications 5 ou 6 **caractérisé** en ce que la résistance est alimentée par un circuit de régulation (9) associé à un capteur de température et avantageusement à une source d'énergie autonome (7) telle que pile ou batterie, prévu pour maintenir la résistance sous tension jusqu'à ce que la température de la dose du produit (2) à l'intérieur de la chambre (5) ait atteint la valeur désirée.

9. Appareil (1) selon l'une quelconque des revendications 7 ou 80 **caractérisé** en ce que des moyens visuels tels que leds (27) et/ou sonores indiquent à l'utilisateur soit que la température du produit (2) est atteinte à l'intérieur de la chambre (5), soit qu'un temps suffisant est écoulé pour permettre l'absorption de la dose ainsi réchauffée, soit encore que les deux sont atteints.

10. Appareil (1) selon l'une quelconques des revendications 5 ou 6 **caractérisé** en ce que l'organe chauffant (10) est une résistance à coefficient positif de température (PTC) associé à une source avantageusement autonome d'énergie (7), procurant une auto-régulation thermique de la température du produit (2) à l'intérieur de la chambre d'expansion (5).

11. Appareil (1) selon l'une quelconque des revendication 4 à 10 **caractérisé** en ce que l'aérosol (3) est couplé par son tube éjecteur (11) de façon étanche avec la chambre d'expansion (5), de telle manière que par une pression verticale comprimant axialement le tube éjecteur (11) dans l'aérosol (3), il entre une dose de produit (2) dans la chambre d'expansion (5).

12. Appareil (1) selon la revendication 11 **caractérisé** en ce que le tube éjecteur (11) est couplé à la chambre (5) par l'intermédiaire d'un joint adaptateur (12) souple pouvant recevoir une large gamme d'aérosol, comportant avantageusement un épaulement intérieur (14b) pour créer un appui vertical lors de la compression tube-éjecteur/aérosol.

13. Appareil (1) selon l'une quelconque des revendications 4 à 12 **caractérisé** en ce que l'embout (16) buccal ou nasal est relié à la chambre d'expansion (5) par un clapet (17) fermé quand ladite chambre (5) est au repos et ouvert quand l'embout (16) est en bonne position dans la bouche, le nez, les oreilles ou autres orifices du corps, le clapet (17) étant automatiquement rappelé en position de fermeture dès le retrait de l'embout (16).

14. Appareil (1) selon la revendication 13 **caractérisé** en ce que l'actionnement du clapet (17) est soit manuel soit obtenu par un épaulement externe (24) venant en appui en périphérie de l'orifice d'inhalation ou d'insufflation qui entraîne, en un mouvement inverse la soupape (19) du clapet (17) lorsque l'embout pénètre dans ledit orifice.

15. Appareil (1) selon l'une quelconque des revendications 11 à 14 **caractérisé** en ce qu'il comprend des moyens permettant d'effectuer simultanément une compression axiale de l'éjecteur (11) de l'aérosol (3) pour délivrer au moins une dose de produit (2) et une mise en fonctionnement des moyens de chauffage de la même dose.

16. Application de l'appareil (1) conforme à l'une quelconque des revendications 4 à 15 à la diffusion des produits bronchodilatateurs sous forme d'aérosol distribués dose par dose en "spray".
